(19) Europäisches Patentamt
European Patent Office
Office européen des brevets

(11) Veröffentlichungsnummer: **0 150 026**
**A2**

(12) **EUROPÄISCHE PATENTANMELDUNG**

(21) Anmeldenummer: 85100279.0

(22) Anmeldetag: 12.01.85

(51) Int. Cl.⁴: **C 07 B 35/02**

(30) Priorität: 16.01.84 HU 15484
27.11.84 HU 15484

(43) Veröffentlichungstag der Anmeldung:
31.07.85 Patentblatt 85/31

(84) Benannte Vertragsstaaten:
AT CH DE FR GB IT LI

(71) Anmelder: **CHINOIN Gyogyszer és Vegyészeti Termékek Gyára RT.**
To utca 1-5
H-1045 Budapest IV(HU)

(72) Erfinder: **Káldor, István, Dipl. Ing. Chem.**
Madách u. 46
Göd(HU)

(72) Erfinder: **Szász, András, Dipl. Ing. Chem.**
Victor Hugo u. 28
H-1032 Budapest(HU)

(72) Erfinder: **Végh, geb. Báji, Ilona**
Kelen J. u. 6
H-1075 Budapest(HU)

(72) Erfinder: **Heizer, József, Dipl. Ing. Chem.**
Vesselényi u. 54
H-1075 Budapest(HU)

(72) Erfinder: **Benedek, geb. Haraszin, Eva**
Vércse u. 18
H-1124 Budapest(HU)

(74) Vertreter: **Lotterhos, Hans Walter, Dr.-Ing.**
Lichtensteinstrasse 3
D-6000 Frankfurt am Main 1(DE)

(54) **Chemisches Verfahren.**

(57) Die Erfindung betrifft ein chemisches Verfahren zur Herstellung von Verbindungen der Formel I

$$R - X - R^1 \qquad (I),$$

($X = -S-, -S-S-, -CO-$,
R und $R^1$ = unabhängig voneinander H, OH;
gegebenenfalls durch mindestens ein OH, Carboxy, $C_{1-6}$-Alkoxycarbonyl, $NH_2$, Acetylamino, $NO_2$, $C_{1-6}$-Alkylamino, $C_{1-6}$-Alkoxy,

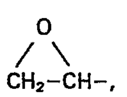

gegebenenfalls durch mindestens ein OH subst, $C_{5-7}$-Cycloalkyl und/oder durch Halogen subst. $C_{1-12}$-Alkyl oder $C_{2-12}$-Alkenyl; $C_{7-10}$-Aralkyl; gegebenenfalls durch mindestens ein $C_{1-4}$-Alkyl, $NO_2$ und/oder Halogen subst. Phenyl oder Naphthyl; 5- oder 6-gliedriger, 1-3 Stickstoffatome enthaltender, gegebenenfalls mit einem Benzolring kondensierter und gegebenenfalls mindestens einen Substituenten enthaltenden Heterocyclus - vorzugsweise 2-Carbomethoxyamino-1H-benzimidazol-5-yl, Pyrrolyl, Indolyl, Imidazolyl, Benzimidazolyl, Pyridyl, Chinolyl, Isochinolyl, 1,2 -, 1,3- oder 1,4-Diazinyl, 1,2,4- oder 1,3,5-Triazinyl, Phthalazinyl oder Chinoxalyl; halogensubst. $C_{1-10}$-Aralkyl oder

$$-(CH_2)_n-\text{Halogen } (n = 1 - 6))$$

durch Reduktion einer Verbindung der Formel II

$$R - A \qquad (II)$$

(A = Chlorfulfonyl, 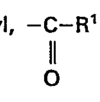 oder 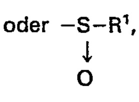,

R und $R^1$ wie oben)
mit einer Schwefelverbindung, die ein S-Atom mit dem Oxydationsgrad (+)4 enthält, der während der Reaktion in den Oxydationsgrad (+)6 überführbar ist oder in saurem Medium zu einer Verbindung mit dem letztgenannten Oxydationsgrad zersetzt werden kann, in Gegenwart von höchstens 0,5 Mol / 1 Mol Ausgangsverbindung der Formel I, vorzugsweise einer katalytischen Menge, elementarem Jod oder einer Verbindung, die in saurem Medium Jodwasserstoff liefert, oder in saurem Medium mit der verwendeten

./...

EP 0 150 026 A2

Croydon Printing Company Ltd.

Schwefelverbindung zu Jodwasserstoff reduzierbar ist, wobei die erhaltene Verbindung der Formel I (wenn $R^1$ H und X $-S-S-$ist) gegebenenfalls in Gegenwart von Cyanidionen mit $C_{1-3}$-Alkyl-Hal (Hal = Br, Cl), in an sich bekannter Weise alkyliert werden kann.

Die Verbindungen der Formel I stellen Zwischenprodukte und Stimulantien dar.

1

## Chemisches Verfahren

Die Erfindung betrifft ein neues chemisches Verfahren zur Herstellung von teilweise bekannten und teilweise neuen Verbindungen der allgemeinen Formel I

$$R - X - R^1 \qquad (I)$$

durch Reduktion von Sulfonsäurechloriden, Sulfoxiden bzw. Halogenverbindungen der allgemeinen Formel II

$$R - A \qquad (II) .$$

In der chemischen Literatur sind bereits mehrere Verfahren zur Herstellung von Disulfiden durch Reduktion von Sulfonsäurechloriden beschrieben worden. So kann z.B. Benzolsulfonsäurechlorid in ätherischer Lösung mit der äquimolaren Menge von Lithiumaluminiumhydrid in das Phenyldisulfid übergeführt werden (J.Org.Chem. 1951, (16) 496); das 3-Nitrobenzolsulfonsäurechlorid kann z.B. mit konzentriertem Jodwasserstoff zu 3-Nitro-phenyldisulfid reduziert werden (Org.Synth. Coll.Vol. 5 (1073)) und aus Benzolsulfonsäurechlorid läßt sich durch Behandlung mit rotem Phosphor in Gegenwart einer katalytischen Menge Jod in einem wäßrig-essigsaurem Medium das Phenyldisulfid herstellen (C.A. 52, 2791 (1958)).

Es ist weiterhin bekannt, daß die Reduktion von Sulfoxiden u.a. mit Zinn(II)chlorid (Synthesis 1973, 206), mit Wasserstoff in Gegenwart eines Palladium/Aktivkohle-Katalysators (Synthesis 1975, 385), mit Natriumborhydrid in Gegenwart von Cobalt(II)chlorid (J.Org.Chem. 19, 613) oder mit Jodwasserstoff (Z.Obs.Khim. 29 3033 = C.A. 54 12096 d) durchgeführt werden kann.

Diese Verfahren weisen mehrere Nachteile auf. In mehreren Fällen sind die Ausbeuten sehr niedrig, die Selektivität ist gering, die Methoden sind nur zur Herstellung einer kleinen Verbindungsgruppe geeignet, zur Erreichung von befriedigenden Ausbeuten sind oft extrem lange Reaktionszeiten erforderlich und in mehreren Fällen ist das Verfahren wegen der notwendigen Reaktionsbedingungen und Reaktanten zur Durchführung im Betrieb ungeeignet.

Bei der Suche nach einem neuen Verfahren das aus der US-PS 4 368 238 bekannte 2-[(Methoxycarbonyl)-amino]-1H-benzimidazol-5-sulfonsäurechlorid zu dem 5,5'-(2-Carbomethoxyamino-benzimidazol)-yl-disulfid zu reduzieren (beide Verbindungen sind in der Synthese des bekannten Anthelminticums Albendazol [2-([Methoxycarbonyl]-amino)-5-propylthio-1H-benzimidazol] als Zwischenprodukte verwendbar (DE-OS 28 20 375).) wurde ein neues Reduktionssystem gefunden, das nicht nur zur selektiven Reduktion des oben genannten Sulfonsäurechlorids, sondern auch zur selektiven Reduktion von vielen anderen Sulfonsäurechloriden, Sulfoxiden bzw. Halogenverbindungen geeignet ist. Die Erfindung wird in der Anwendung dieses neuen Reduktionssystems auf die Herstellung von neuen und von bekannten Verbindungen gesehen.

Gegenstand der vorliegenden Erfindung ist ein chemisches Verfahren zur Herstellung von Verbindungen der allgemeinen Formel I

$$R - X - R^1 \qquad (I),$$

worin

X eine Thio-, Dithio- oder Carbonylgruppe,

R und $R^1$ unabhängig voneinander Wasserstoff, Hydroxy; gegebenenfalls durch mindestens ein Hydroxy, Carboxy, $C_{1-6}$-Alkoxycarbonyl, Amino, Acetylamino, Nitro, $C_{1-6}$-Alkylamino, $C_{1-6}$-Alkoxy, 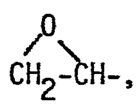, gegebenenfalls durch mindestens ein Hydroxy substituiertes $C_{5-7}$-Cycloalkyl und/oder durch Halogen substituiertes geradkettiges oder verzweigtes $C_{1-12}$-Alkyl oder $C_{2-12}$-Alkenyl;
$C_{7-10}$-Aralkyl;

gegebenenfalls durch mindestens ein $C_{1-4}$-Alkyl, Nitro und/ oder Halogen substituiertes Phenyl oder Naphthyl oder einen 5- oder 6-gliedrigen, 1-3 Stickstoffatome enthaltenden gegebenenfalls mit einem Benzolring kondensierten und gegebenenfalls mindestens einen Substituenten tragenden heterocyclischen Ring - vorzugsweise 2-Carbomethoxyamino-1H-benzimidazol-5-yl, Pyrrolyl, Indolyl, Imidazolyl, Benzimidazolyl, Pyridyl, Chinolyl, Isochinolyl, 1,2-, 1,3- oder 1,4-Diazinyl, 1,2,4- oder 1,3,5-Triazinyl, Phthalazinyl oder Chinoxalyl;

halogensubstituiertes $C_{7-10}$-Aralkyl oder eine Gruppe der allgemeinen Formel

$$-(CH_2)_n\text{-Halogen},$$

in der n = 1-6 ist,

bedeuten,

das dadurch gekennzeichnet ist, daß man eine Verbindung der allgemeinen Formel II

$$R - A \qquad (II),$$

worin

A eine Chlorsulfonylgruppe oder eine Gruppe der allgemeinen Formel

$-\underset{\underset{O}{\|}}{C}-R^1$ oder $-\underset{\underset{O}{\downarrow}}{S}-R^1$ bedeutet und

R und $R^1$ die oben angegebene Bedeutung haben,

mit einer Schwefelverbindung reduziert, die ein Schwefelatom enthält, das den Oxydationsgrad von (+)4 aufweist und während der Reaktion in den Oxydationsgrad (+)6 überführbar ist, oder in saurem Medium zu einer Verbindung mit dem letztgenannten Oxydationsgrad zersetzt werden kann,

wobei die Reduktion in Gegenwart von 0,05 - 0,1, höchstens 0,5 Mol / 1 Mol der Ausgangsverbindung der allgemeinen Formel II, vorzugsweise einer katalytischen Menge an elementarem Jod oder einer Verbindung durchgeführt wird, die in saurem Medium Jodwasserstoff liefert, oder in saurem Medium mit der verwendeten Schwefelverbindung zu Jodwasserstoff reduzierbar ist, und

wobei gewünschtenfalls die erhaltene Verbindung der allgemeinen Formel I, wenn R die oben angegebene Bedeutung hat, $R^1$ Wasserstoff und X die Dithiogruppe bedeutet, gegebenenfalls in Gegenwart von Cyanidionen mit einer Verbindung der allgemeinen Formel

$$R^1 - Hal,$$

worin

$R^1$ $C_{1-3}$-Alkyl und

Hal Brom oder Chlor ist,

in an sich bekannter Weise alkyliert wird.

Die Reduktion kann bei einer Temperatur im Bereich von 10 bis 150°C, vorzugsweise von 40 bis 120°C, insbesondere von 40 bis 60°C, durchgeführt werden.

Als Reaktionsmedium können inerte Lösungsmittel oder deren Gemische dienen. Als zweckmäßig haben sich 1 - 50 Gew.% Wasser enthaltende Mineralsäuren oder organische Säuren oder deren Gemische erwiesen. Als organische Säure kann z.B. Essigsäure oder Ameisensäure und als Mineralsäure kann Salzsäure verwendet werden.

Einige Beispiele für Schwefelverbindungen mit dem Oxydationsgrad (+)4 sind Schwefeldioxyd, Alkalimetall- und Erdalkalimetallsulfite, -bisulfite oder -thiosulfate und
einige Beispiele für Verbindungen, die in saurem Medium zu Jodwasserstoff reduzierbar sind, sind Alkalimetalljodide.

Die Ausgangsverbindungen der allgemeinen Formel II sind teilweise bekannte Handelsprodukte, teilweise sind sie durch an sich bekannte, in der organischen Chemie übliche Verfahren herstellbar (J.Chem.Soc.Co., 1981, (6) 295). Die Herstellung des 2-Carbomethoxy-amino-1H-benzimidazol-5-sulfonsäurechlorids ist in Beispiel 1 der US-PS 4 368 328 beschrieben. Einige Vertreter der Verbindungen der allgemeinen Formel I sind wertvolle Zwischenprodukte der Albendazol-Synthese, andere Verbindungen der allgemeinen Formel I weisen wertvolle immunstimulierende Eigenschaften auf (DE-OS 32 43 352).

Die nachfolgend aufgeführten Verbindungen der allgemeinen Formel I, die nach dem erfindungsgemäßen Verfahren herstellbar und in der DE-OS 32 43 352 beschrieben sind, üben eine besonders günstige immunstimulierende Wirkung aus:

a) $HO-(CH_2)_2-S-S-(CH_2)_2-OH$;

b) $HO-(CH_2)_3-S-S-(CH_2)_3-OH$;

c) $Cl-CH_2-CH-CH_2-S-S-CH_2-CH-CH_2-Cl$;
OH OH

d) 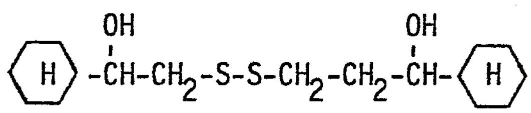 ;

e) $CH_3-(CH_2)_3-CH-CH_2-S-S-CH_2-CH-(CH_2)_3-CH_3$;
OH OH

f) $CH_3-CH-CH_2-S-S-CH_2-CH-CH_3$;
OH OH

g) $CH_3O-(CH_2)_2-S-S-(CH_2)_2-OCH_3$;

h) $CH_3-(CH_2)_3-CH-CH_2-S-S-CH_2-CH-(CH_2)_3-CH_3$;
OH OH

i) $CH_3-CH-CH_2-S-S-CH_2-CH-CH_3$
OH OH

j) $HO-CH_2-CH-CH_2-S-S-CH_2-CH-CH_2-OH$;
OH OH

k) $CH_3-O-CH_2-C(CH_3)_2-S-S-C(CH_3)_2-CH_2-OH$;

l) 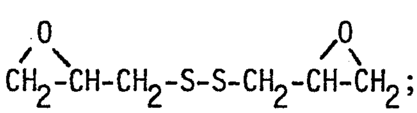;

m) $CH_3-NH-(CH_2)_2-S-S-(CH_2)_2-NH-CH_3$:

n) $i-C_3H_7-S-S-(CH_2)_2-OH$;

o) $t-C_4H_9-S-S-(CH_2)_2-OH$;

$CH_3$ OH

p) $CH_3-CH_2-CH-S-S-CH_2-CH-CH_3$;

OH

q) $i-C_3H_7-S-S-CH_2-CH-CH_3$;

OH

r) $t-C_4H_9-S-S-CH_2-CH-CH_3$:

s) $CH_3-CH_2-CH-S-S-(CH_2)_2)OH$;

$CH_3$

t) $i-C_3H_7-CH(i-C_3H_7)-S-S-(CH_2)_2-OH$;

u) 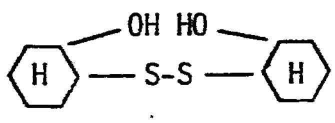

v) 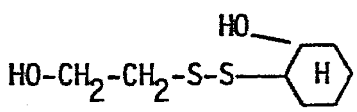

Das neue Reduktionsverfahren der Erfindung weist folgende Vorteile auf: Mit Hilfe des Verfahrens der Erfindung können aus Sulfiden bzw. Sulfonsäuren symmetrische oder gemischte Thioäther und Disulfide hergestellt werden, und ein der Aldehyd- bzw. Ketocarbonylgruppe benachbartes Halogenatom kann gegen ein Wasserstoffatom ausgetauscht werden.

Gemäß Erfindung erhaltene Disulfide, in denen $R^1$ Wasserstoff bedeutet, können durch Umsetzung mit $C_{1-3}$-Alkylhalogeniden in Gegenwart von vorzugsweise Alkalicyaniden, wie Natrium- oder Kaliumcyanid, in an sich bekannter Weise alkyliert werden.

Zur Herstellung asymmetrischer Thioäther können auch verschiedene Ausgangsverbindungen der allgemeinen Formel R - A, z.B. verschiedene Chlorsulfonsäuren, gleichzeitig gemäß Erfindung reduziert werden.

Die Selektivität der Methode ist hoch; das Verfahren der Erfindung kann u.a. auch in Gegenwart einer alkoholischen Hydroygruppe, einer

Aldehyd-, Keton- oder Estercarbonylgruppe, einer Carbonsäure- oder Nitrogruppe bzw, gegebenenfalls aktivierten Doppelbindung durchgeführt werden.

Weitere Einzelheiten des Verfahrens der Erfindung sind den nachstehenden Beispielen zu entnehmen, ohne den Schutzumfang auf diese Beispiele einzuschränken.

Beispiel 1

Herstellung von p-Tolyl-disulfid.

19 g (0,1 Mol) p-Toluolsulfonsäurechlorid löst man in 100 ml Essigsäure, gibt der Lösung 1,5 g (0,01 Mol) Natriumjodid zu und rührt das Reaktionsgemisch bis zur vollständigen Auflösung des Jodids (etwa 5 Minuten). Das Reaktionsgemisch nimmt eine dunkelbraune Farbe an. Sodann tropft man dem Reaktionsgemisch langsam eine 30 %ige wäßrige Natriumbisulfitlösung mit solcher Geschwindigkeit zu, daß - unter Einwirkung des Bisulfits - die Jodfarbe des Reaktionsgemisches verschwindet. Während der Zugabe wird das Reaktionsgemisch auf 60°C erwärmt (innerhalb von etwa 30 Minuten). Die Reaktion wird bei dieser Temperatur so lange fortgesetzt, bis die Jodfarbe nicht mehr auftritt. Nach Beendigung der Umsetzung verdünnt man das Reaktionsgemisch mit 50 ml Wasser und stellt es mit 20 %iger Natriumhydroxidlösung auf pH 4 ein. Das Reaktionsgemisch extrahiert man 3 mal mit je 80 ml n-Hexan, vereinigt die Hexanphasen, trocknet sie über wasserfreiem Natriumsulfat, engt sie unter Atmosphärendruck ein, entfernt aus dem Rückstand die Lösungsmittelspuren bei 70°C unter vermindertem Druck und bringt das Produkt bei 20°C zur Kristallisation. Es werden 11,9 g der im Titel genannten Verbindung erhalten.

Ausbeute 97 %. F.: 43 - 45°C 1 bar

| | | | | |
|---|---|---|---|---|
| $^{1}$H-NMR: ($CDCl_3$): aromatisch | 7,35 ppm | (d) | 2H |
| | 7,05 ppm | (d) | 2H |
| $CH_3$ | 2,8 ppm | (s) | 3H |

Beispiel 2

Herstellung von Phenyldisulfid.

Man verfährt wie in Beispiel 1, jedoch mit dem Unterschied, daß man anstatt von p-Toluolsulfonsäurechlorid als Ausgangsverbindung Benzolsulfonsäurechlorid verwendet. Es werden 10 g der im Titel genannten Verbindung erhalten.

Ausbeute 93 %. F.: 58 - 60°C 1 bar

$^{1}$H-NMR: ($CDCl_3$): aromatisch 7,6 - 7,0 ppm (m)

Beispiel 3

Herstellung von Methyldisulfid.

Man verfährt wie in Beispiel 1, jedoch mit dem Unterschied, daß man als Ausgangsverbindung Methansulfonsäurechlorid verwendet und bei der Aufarbeitung des Reaktionsgemisches den pH-Wert nicht auf 4 sondern auf 8 einstellt. Das Reaktionsgemisch wird 3 mal mit je 50 ml Äther extrahiert; die ätherischen Extrakte werden vereinigt, getrocknet und fraktioniert, wobei man 2,85 g der im Titel genannten Verbindung erhält.

Ausbeute 61 %. Siedepunkt: 107 - 109°C 1 bar, $n_D^{25}$ = 1,5249.

Beispiel 4

Herstellung von 5,5'-(2-Carbomethoxyamino-benzimidazol)-yl-disulfid.

Man verfährt wie in Beispiel 1, jedoch mit dem Unterschied, daß man als Ausgangsverbindung anstatt von p-Toluol-sulfonsäurechlorid 2-Carbomethoxyamino-benzimidazol-5-sulfonsäurechlorid verwendet, bei der Aufarbeitung des Reaktionsgemisches die Hexan-Extraktion wegläßt, den pH-Wert auf 4 einstellt, das Reaktionsgemisch bei 50°C filtriert und die erhaltenen Kristalle 3 mal mit je 10 ml heißem Wasser wäscht, so daß sie frei von Sulfationen sind. Das Produkt wird unter vermindertem Druck bei 100°C getrocknet. Es werden 18,2 g der im Titel genannten Verbindung erhalten.

Ausbeute 82 %. F.: 323 - 325°C.

| | | | | |
|---|---|---|---|---|
| $^1$H-NMR: (in TFA-$d_1$): | $C_7$-H | 7,8 ppm | s | 1H |
| | $C_6$-H | 7,6 ppm | s | 2H |
| | $C_4$-H | 7,6 ppm | s | 2H |
| | $OCH_3$ | 4,0 ppm | s | 3H |

Beispiel 5

Herstellung von 2/(Methoxycarbonyl)-amino/-5-propylthio-1H-benzimidazol.

36 g des nach Beispiel 4 hergestellten 5,5'-(2-Carbomethoxyamino-benzimidazol)-yl-disulfids löst man in einem Gemisch von 30 g Kaliumhydroxid, 25 ml Propylbromid, 7 g Kaliumcyanid, 250 ml Wasser und 500 ml Aceton und rührt das Reaktionsgemisch bei 20 - 25°C 20 Stunden. Nach Zugabe von 35 g Natriumbicarbonat wird das ausgeschiedene Produkt abfiltriert, mit 200 ml 50 Vol.%igem wäßrigen Aceton und dann mit 200 ml Wasser gewaschen. Es werden 33 g der im Titel genannten Verbindung erhalten.

Ausbeute 75 %. F.: 210 - 212°C.

Beispiel 6

Herstellung von p-Tolyl-1-propenyl-thioäther.

10 Millimol p-Tolyl-cis-trans-1-propenyl-sulfoxid löst man in 10 ml Essigsäure. gibt dann 0,2 g Natriumjodid zu, erhöht die Temperatur des Gemisches auf 50°C und leitet die Reaktion durch Zugabe von 2 Tropfen konzentrierter Salzsäure ein. Das gebildete Jod wird durch kontinuierliche Zugabe einer 30 %igen Natriumbisulfitlösung reduziert, und nach dem Verschwinden der Jodfarbe gibt man dem Reaktionsgemisch 3 - 5 Tropfen konzentrierter Salzsäure zu. Die konzentrierte Salzsäure und die Natriumbisulfitlösung werden alternativ nacheinander so lange zugegeben, bis bei der Zugabe der nächsten Salzsäureportion die Jodfarbe nicht mehr auftritt.

Das Reaktionsgemisch wird mit 10 ml Wasser verdünnt, der pH-Wert auf 8 eingestellt und das gebildete Disulfid 3 mal mit je 15 ml n-Hexan extrahiert. Die vereinigten Hexanphasen werden über Natriumsulfat getrocknet und eingeengt. Man erhält 2,1 g der im Titel genannten Verbindung.

Ausbeute 85 %. F.: 43 - 45°C.

Beispiel 7

Herstellung von Methyl-phenyl-thioäther (Thioanisol).

Man verfährt wie in Beispiel 6, jedoch mit dem Unterschied, daß man als Ausgangsverbindung anstatt von p-Tolyl-cis-trans-1-propenyl-sulfoxid Methyl-phenyl-sulfoxid verwendet und den lösungsmittelfreien Destillationsrückstand fraktioniert. Siedepunkt: 181°C. (1 bar). Es werden 1,1 g der im Titel genannten Verbindung erhalten.

Ausbeute 88 %. $n_D^{25}$ = 1,5850

| | | | | |
|---|---|---|---|---|
| $^1H$-NMR: ($CDCl_3$): | aromatisch | 7,4 ppm | (s) | 5H |
| | $-CH_3$ | 2,47 ppm | (s) | 3H |

<u>Beispiel 8</u>

Herstellung von p-Tolyl-n-propyl-thioäther.

Man verfährt wie in Beispiel 6, jedoch mit dem Unterschied, daß man als Ausgangsverbindung n-Propyl-p-tolyl-sulfoxid verwendet und den Destillationsrückstand fraktioniert. Es werden 1,4 g der im Titel genannten Verbindung erhalten.

Ausbeute 92 %. Siedepunkt: 78 -80°C / 266 Pa, $n_D^{25}$ = 1,5450

| | | | | |
|---|---|---|---|---|
| $^1H$-NMR: ($CDCl_3$) | aromatisch | 7,25 ppm | (d) | 2H |
| | | 7,05 ppm | (d) | 2H |
| | $SCH_2$ | 2,85 ppm | (t) | 2H |
| | $CH_3$ | 2,3 ppm | (s) | 3H |
| | $\underline{CH_2}CH_3$ | 1,65 ppm | (m) | 2H |
| | $CH_2\underline{CH_3}$ | 1,0 ppm | (t) | 3H |

<u>Beispiel 9</u>

Herstellung von p-Tolyl-allyl-thioäther.

Man verfährt wie in Beispiel 6, jedoch mit dem Unterschied, daß man als Ausgangsverbindung p-Tolyl-allyl-sulfoxid verwendet. Es werden 0,65 g der im Titel genannten Verbindung erhalten.

Ausbeute 39 %. Siedepunkt: 70 -73°C / 266 Pa, $n_D^{25}$ = 1,5710

| | | | | | |
|---|---|---|---|---|---|
| $^1H$-NMR: ($CDCl_3$): | aromatisch | 7,25 ppm | (d) | 2H | |
| | | 7,05 ppm | (d) | 2H | |
| | -CH= | 6,1 - 5,6 ppm | (m) | 1H | $^3J_{CH, = CH_A}$ = 9,6<br>$^3J_{CH=CH_B}$ = 17 Hz |
| | $=CH_2$ | 5,1 ppm | (dd) | 2H | $^2J_{CH_A=CH_B}$ = 1 |
| | $SCH_2$ | 3,5 ppm | (d) | 2H | $^3J_{CH_2, CH}$ = 6m8 Hz<br>$^4J_{CH_2, =CH_2}$ = 0,8 Hz |
| | $-CH_3$ | 2,3 ppm | (s) | 3H | |

<u>Beispiel 10</u>

Herstellung von Phenyl-3-butenyl-thioäther.

Man verfährt wie in Beispiel 6, jedoch mit dem Unterschied, daß man

als Ausgangsverbindung Phenyl-3-butenyl-sulfoxid verwendet. Es werden 1,2 g der im Titel genannten Verbindung erhalten.
Ausbeute 72 %.

| | | | | |
|---|---|---|---|---|
| $^1$H-NMR: ($CDCl_3$): | aromatisch | 7,5 - 7,0 ppm | (m) | 5H |
| | -CH= | 6,15 - 5,6 ppm | (m) | 1H |
| | $=CH_2$ | 5,1 ppm | (dd) | 2H |
| | $-SCH_2$ | 3,0 ppm | (t) | 2H |
| | | | $^3J_{CH_2,\ H_2}$ = 8 Hz | |
| | $-CH_2CH=$ | 2,85 ppm | (q) | 2H |

Beispiel 11

Herstellung von p-Tolyl-benzyl-thioäther.

Man verfährt wie in Beispiel 6, jedoch mit dem Unterschied, daß man als Ausgangsverbindung p-Tolyl-benzyl-sulfoxid verwendet. Es werden 1,9 g der im Titel genannten Verbindung erhalten.
Ausbeute 88,7 %. F.: 43 - 44°C

| | | | | |
|---|---|---|---|---|
| $^1$H-NMR: ($CDCl_3$): | monosubstituiert aromatisch | 7,5 ppm | (s) | 5H |
| | disubstituiert aromatisch | 7,1 ppm | (dd) | 4H |
| | $-CH_2-$ | 4,05 ppm | (s) | 2H |
| | $-CH_3-$ | 2,3 ppm | (s) | 3H |

Beispiel 12

Herstellung von Phenyl-ß-phenyläthyl-thioäther.

Man verfährt wie in Beispiel 6, jedoch mit dem Unterschied, daß man als Ausgangsverbindung Phenyl-ß-phenyläthyl-sulfoxid verwendet. Es werden 1,6 g der im Titel genannten Verbindung erhalten.
Ausbeute 75 %. $n_D^{25}$ = 1,6101.

| | | | | |
|---|---|---|---|---|
| $^1$H-NMR: ($CDCl_3$): | aromatisch | 7,25 - 7,0 ppm | (m) | 10H |
| | $-CH_2CH_2$ | 3,0 ppm | (m) | 4H |

Beispiel 13

Herstellung von n-Propyl-5-(2-carbomethoxyamino-benzimidazol)-yl-thioäther.

Man verfährt wie in Beispiel 6, jedoch mit dem Unterschied, daß man

als Ausgangsverbindung n-Propyl-5-(2-carbomethoxyamino-benzimidazol)-yl-sulfoxid verwendet. Bei der Aufarbeitung des Reaktionsgemisches läßt man die Hexanextraktion weg, filtriert aus dem neutralisierten Reaktionsgemisch (pH 7) die Kristalle ab und wäscht sie 3 mal mit je 10 ml Wasser. Es werden 2,1 g der im Titel genannten Verbindung erhalten.

Ausbeute 79 %. F.: 210 - 212°C.

| | | | | |
|---|---|---|---|---|
| $^1$H-NMR: ($CDCl_3$): | -NH | 11,6 ppm | (s) | 1H |
| | $C_7$-H | 7,45 ppm | (s) | 1H |
| | $C_4$-H | 7,35 ppm | (d) | 1H |
| | $C_6$-H | 7,1 ppm | (d) | 1H |
| | O-$CH_3$ | 3,75 ppm | (s) | 3H |
| | S-$CH_2$ | 2,75 ppm | (t) | 2H |
| | -$CH_2CH_3$ | 1,55 ppm | (m) | 2H |

Beispiel 14

Herstellung von Phenyl-acetaldehyd.

100 Millimol $\alpha$-Brom-$\alpha$-phenyl-acetaldehyd löst man in 100 ml Essigsäure, gibt der Lösung 0,3 g (2 Millimol) Natriumjodid zu und rührt das Reaktionsgemisch so lange, bis das Jodid vollständig in Lösung geht. Das in der Reaktion gebildete Jod wird mit einer 30 %igen Kaliumbisulfitlösung kontinuierlich reduziert (Bisulfitlösungsaufnahme 4,5 ml). Wenn die Farbe des Jods nicht mehr auftritt, wird das Gemisch mit einer 10 %igen Natriumhydroxidlösung auf pH 4 eingestellt. Das Reaktionsgemisch wird 3 mal mit je 20 ml Äther extrahiert, die ätherischen Phasen werden vereinigt, über Natriumsulfat getrocknet und eingeengt. Der Rückstand wir unter vermindertem Druck fraktioniert. Es werden 4,5 g der im Titel genannten Verbindung erhalten.

Ausbeute 38 %. Siedepunkt: 108°C / 660 Pa

| | | | | |
|---|---|---|---|---|
| $^1$H-NMR: ($CDCl_3$): | CHO | 9,75 ppm | (t) | 1H |
| | aromatisch | 7,5 - 7,0 ppm | (m) | 5H |
| | -$CH_2$- | 3,65 ppm | (d) | 2H |

$^3J_{CH_2,\ CHO}$ = 2,3 Hz

Beispiel 15

Herstellung von p-Nitro-acetophenon.

Man verfährt wie in Beispiel 14, jedoch mit dem Unterschied, daß man als Ausgangsverbindung anstatt von α-Brom- α-phenyl-acetaldehyd Brom-methyl-p-nitro-phenylketon verwendet. Bei der Aufarbeitung des Reaktionsgemisches führt man nicht die in Beispiel 14 beschriebene Extraktion durch, sondern fällt das Produkt mit 50 ml Wasser aus, filtriert es ab und wäscht es 3 mal mit je 20 ml Wasser. Es werden 16,1 g der im Titel genannten Verbindung erhalten.

Ausbeute 98 %. F.: 78 - 79°C.

| | | | | |
|---|---|---|---|---|
| $^1$H-NMR: ($CDCl_3$): aromatisch | 8,3 ppm | (d) | 2H |
| | 8,1 ppm | (d) | 2H |
| $-CH_3$ | 2,6 ppm | (s) | 3H |

Beispiel 16

Herstellung von p-Tolyl-5-(2-carbomethoxyamino-1H-benzimidazol)-yl-disulfid.

1,9 g (10 Millimol) p-Toluolsulfonsäurechlorid und 2,9 g (10 Millimol) 2-[(Methoxycarbonyl)-amino]-1H-benzimidazol-5-sulfonsäurechlorid werden in 20 ml Essigsäure gelöst und der Lösung 0,3 g (2 Millimol) Natriumjodid zugegeben. Das Gemisch wird so lange gerührt, bis sich das Jodid auflöst. Die Lösung nimmt eine dunkle Jodfarbe an. Das in der Reaktion gebildete Jod wird durch Zugabe einer 30 %igen Natriumbisulfitlösung kontinuierlich reduziert. Die Temperatur des Reaktionsgemisches erhöht man inzwischen innerhalb von 30 Minuten langsam auf 70°C. Am Ende der Reaktion erscheint die Jodfarbe nicht mehr. Die Natriumbisulfitlösungsaufnahme beträgt 20 ml. Das erhaltene Reaktionsgemisch wird mit einer 20 %igen Natriumhydroxidlösung auf pH 4 eingestellt, die ausgeschiedenen Kristalle werden filtriert und 3 mal mit je 20 ml Wasser gewaschen. Das Produkt wird auf einer Kieselgel G (Stahl) Säule (Länge 25 cm; Durchmesser 2,5 cm) unter einem Überdruck von 2 bar chromatographiert und mit einer 9 : 1 Mischung von Chloroform und Essigsäure eluiert. Der $R_f$-Wert der im Titel genannten Verbindung beträgt 0,62. Durch Einengen des Eluats wird das gewünschte Produkt in einer Ausbeute von 40 % erhalten. F.: 225 - 230°C (Zersetzung).

| | | | | |
|---|---|---|---|---|
| $^1H$-NMR: ($DMSO-d_6$): -NH- | 12,65 | ppm | (s) | 1H |
| aromatisch | 7,6 - 7,1 | ppm | (m) | 7H |
| $-O-CH_3-$ | 3,75 | ppm | (s) | 3H |
| $-CH_3$ | 2,3 | ppm | (s) | 3H |

Patentansprüche

1. Chemisches Verfahren zur Herstellung von Verbindungen der allgemeinen Formel I

$$R - X - R^1 \qquad (I),$$

worin

X eine Thio-, Dithio- oder Carbonylgruppe und

R und $R^1$ unabhängig voneinander Wasserstoff, Hydroxy; gegebenenfalls durch mindestens ein Hydroxy, Carboxy, $C_{1-6}$-Alkoxycarbonyl, Amino, $C_{1-4}$-Acylamino, Nitro, $C_{1-6}$-Alkylamino, $C_{1-6}$-Alkoxy, 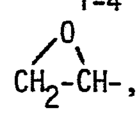, gegebenenfalls durch mindestens ein Hydroxy substituiertes $C_{5-7}$-Cycloalkyl und/oder durch Halogen substituiertes geradkettiges oder verzweigtes $C_{1-12}$-Alkyl oder $C_{2-12}$-Alkenyl;
$C_{7-10}$-Aralkyl;
gegebenenfalls durch mindestens ein $C_{1-4}$-Alkyl, Nitro und/oder Halogen substituiertes Phenyl oder Naphthyl;
einen 5- oder 6-gliedrigen, 1-3 Stickstoffatome enthaltenden, gegebenenfalls mit einem Benzolring kondensierten und gegebenenfalls mindestens einen Substituenten tragenden heterocyclischen Ring - vorzugsweise 2-Carbomethoxyamino-1H-benzimidazol-5-yl, Pyrrolyl, Indolyl, Imidazolyl, Benzimidazolyl, Pyridyl, Chinolyl, Isochinolyl, 1,2-, 1,3- oder 1,4-Diazinyl-, 1,2,4- oder 1,3,5-Triazinyl, Phthalazinyl oder Chinoxalyl;

halogensubstituiertes $C_{7-10}$-Aralkyl oder eine Gruppe der allgemeinen Formel
$-(CH_2)_n$-Halogen ,
in der
n = 1 - 6 ist, bedeuten,

dadurch gekennzeichnet, daß man eine Verbindung der allgemeinen Formel II

$$R - A \qquad (II) ,$$

worin

A eine Chlorsulfonylgruppe oder eine Gruppe der allgemeinen Formel
$-\underset{\overset{\|}{O}}{C}-R^1$ oder $-\underset{\overset{\downarrow}{O}}{S}-R^1$ bedeutet und

R und $R^1$ die oben angegebene Bedeutung haben,

mit einer Schwefelverbindung reduziert, die ein Schwefelatom enthält, das den Oxydationsgrad von (+)4 aufweist und während der Reaktion in den Oxydationsgrad von (+)6 überführbar ist, oder die in saurem Medium zu einer Verbindung mit dem letztgenannten Oxydationsgrad zersetzt werden kann,

wobei die Reduktion in Gegenwart von höchstens 0,5 Mol / 1 Mol der Ausgangsverbindung der allgemeinen Formel II, vorzugsweise einer katalytischen Menge an elementarem Jod oder einer Verbindung durchgeführt wird, die in saurem Medium Jodwasserstoff liefert, oder in saurem Medium mit der verwendeten Schwefelverbindung zu Jodwasserstoff reduzierbar ist, und

wobei gewünschtenfalls die erhaltene Verbindung der allgemeinen Formel I, wenn R die oben angegebene Bedeutung hat, $R^1$ Wasserstoff und X die Dithiogruppe bedeutet, gegebenenfalls in Gegenwart von Cyanid-ionen mit einer Verbindung der allgemeinen Formel

$$R^1\text{-Hal} ,$$

worin

$R^1$ $C_{1-3}$-Alkyl und

Hal Brom oder Chlor ist,

in an sich bekannter Weise alkyliert wird.

2. Verfahren zur Herstellung von Verbindungen der allgemeinen Formel I

$$R - X - R^1 \qquad (I),$$

worin

R eine gegebenenfalls durch mindestens ein $C_{1-4}$-Alkyl, Nitro, Amino, $C_{1-4}$-Alkylamino, $C_{1-4}$-Acylamino, $C_{2-4}$-Alkoxycarbonylamino, $C_{1-4}$-Acyl und/oder Halogen substituierte aromatische Gruppe oder eine gegebenenfalls durch ein Hydroxy, Carboxy, $C_{2-4}$-Alkoxycarbonyl, Amino, Acetylamino oder Nitro substituierte $C_{1-12}$-Alkylgruppe,

X eine Thio- oder Dithiogruppe und

$R^1$ Wasserstoff, Hydroxy oder $C_{1-6}$-Alkoxy bedeuten,

dadurch gekennzeichnet, daß man eine Verbindung der allgemeinen Formel II

$$R - A \qquad (II),$$

worin

A eine Chlorsulfonylgruppe ist und

R die oben angegebene Bedeutung hat,

mit einer Jodverbindung und einer Schwefelverbindung, die einen Oxydationsgrad von (+)4 aufweist oder in saurem Medium zu einem solchen Oxydationsgrad zersetzt wird, reduziert und gewünschtenfalls die so erhaltene Verbindung gegebenenfalls in Gegenwart eines Cyanids alkyliert.

3. Verfahren nach Anspruch 1 oder 2, dadurch gekennzeichnet, daß man die Umsetzung in einem Gemisch von Wasser und einer Mineralsäure und/oder organischen Säure durchführt.

4. Verfahren nach Anspruch 3, dadurch gekennzeichnet, daß man als Mineralsäure Schwefelsäure oder Salzsäure und als organische Säure Essigsäure verwendet.

5. Verfahren nach Anspruch 1 oder 2, dadurch gekennzeichnet, daß man als Schwefelverbindung des Oxydationsgrades (+)4 ein Alkalimetallbisulfit, -sulfit oder -thiosulfat verwendet.

6. Verfahren nach Anspruch 1 oder 2, dadurch gekennzeichnet, daß man als Verbindung, die in saurem Medium Jodwasserstoff liefert, ein Alkalimetalljodid - vorzugsweise Natriumjodid - verwendet.

7. Verfahren nach Anspruch 1 oder 2, dadurch gekennzeichnet, daß man das elementare Jod oder die in saurem Medium Jodwasserstoff liefernde Verbindung in einer Menge von 0,05 - 0,1 Mol - auf 1 Mol der Ausgangsverbindung der allgemeinen Formel II bezogen - verwendet.

8. Verfahren nach Anspruch 1 oder 2, dadurch gekennzeichnet, daß man die Umsetzung bei einer Temperatur von 10 - 150, vorzugsweise von 40 - 120°C, durchführt.

9. Verbindungen der allgemeinen Formel I

$$R - X - R^1,$$

worin
R, X und $R^1$ die im Anspruch 1 angegebene Bedeutung haben und R und $R^1$ voneinander verschieden sind.